# FASCICULE DE BREVET EUROPEEN

(11) **EP 1 430 883 B1**
(45) Date de publication et mention de la délivrance du brevet: **09.11.2005**
(21) Numéro de dépôt: 03292981.2
(22) Date de dépôt: 28.11.2003
(51) Int. Cl.: A61K 7/48

(54) **Utilisation cosmétique de dérivés de l'acide ascorbique comme agents blanchissants de la peau ou des cheveux**
Kosmetische Vewendung von Ascorbinsäurederivaten als Haut- oder Haar-Aufheller
Cosmetic use of ascorbic acid derivatives as whitening agents for skin or hair

(30) Priorité: 18.12.2002 FR 0216109
(43) Date de publication de la demande: 23.06.2004
(73) Titulaire: L'OREAL, 75008 Paris (FR)
(72) Inventeur: Dalko, Maria, 91190 Gif S/Yvette (FR); Cavezza, Alexandre, 93290 Tremblay-en-France (FR)
(74) Mandataire: Kromer, Christophe

(56) Documents cités:
- EP-A- 0 664 290
- EP-A- 0 710 478
- EP-A- 1 145 710

## Description

La présente invention a trait à l'utilisation d'esters de l'acide ascorbique dans une composition, notamment cosmétique, lesdits dérivés et/ou ladite composition étant destinés à blanchir la peau humaine, les poils ou les cheveux.

L'invention a également trait à l'utilisation d'esters de l'acide ascorbique pour la préparation d'une composition, notamment dermatologique, destinée à dépigmenter la peau humaine, les poils ou les cheveux.

La couleur de la peau humaine est fonction de différents facteurs et notamment des saisons de l'année, de la race et du sexe; elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine; lorsque les poils ou les cheveux sont foncés, certaines personnes désirent voir ceux-ci plus clairs. Ceci est particulièrement intéressant pour les poils qui sont moins visibles lorsqu'ils sont clairs que lorsqu'ils sont foncés.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

Le mécanisme de formation de la pigmentation de la peau, c'est-à-dire de la formation de la mélanine est particulièrement complexe et fait intervenir schématiquement les principales étapes suivantes :
Tyrosine ---> Dopa ---> Dopaquinone ---> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. Ils naissent du réticulum endoplasmique sous formes de vacuoles sphériques appelées prémélanosomes. Les prémélanosomes contiennent un substrat protéinique amorphe, mais pas d'enzymes mélanogènes. Au cours de la maturation du prémélanosome, le substrat amorphe s'organise en une structure fibrillaire orientée dans l'axe longitudinal du mélanosome. On distingue quatre stades du développement du mélanosome correspondant à l'intensité de la mélanisation. La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes. Les prolongements dendritiques des mélanocytes jouent donc un rôle essentiel dans le transfert de la mélanine.

Bien que le taux de mélanine varie d'une population à une autre, la quantité de tyrosinase ne varie pas significativement et le taux d'ARN messagers de la tyrosinase est identique dans des peaux blanches ou noires. Les variations dans la mélanogénèse sont donc dues à des variations soit de l'activité de la tyrosinase, soit de la capacité des kératinocytes à phagocyter les mélanosomes, soit encore du nombre des prolongements dendritiques.

Une substance est reconnue comme dépigmentante si elle agit directement sur la vitalité des mélanocytes épidermiques où se déroule la mélanogénèse, par exemple en modifiant la morphologie des prolongements dendritiques, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé est instable en solution, ce qui complique quelque peu la fabrication de la composition.

L'activité de l'acide ascorbique ou de certains de ses dérivés sur la pigmentation est connue depuis de nombreuses années. Dans le but d'expliquer leur action sur la mélanogénèse, un effet inhibiteur sur la tyrosinase a été mis en évidence pour l'acide ascorbique (J. Soc. Cosmet. Chem., 42, 1991, p. 361-368). Récemment, une étude a également montré que l'ascorbyl phosphate de magnésium pouvait avoir un effet sur le transfert de la mélanine et diminuer la dendricité des mélanocytes (Pigment. Cell. Res., 13, 2000, p. 89-98 et p. 190-192), induisant ainsi un effet dépigmentant. Cet effet est comparable à celui pouvant être obtenu avec l'acide férulique.

La demande EP-A-0710478 décrit des compositions cosmétiques blanchissantes contenant du palmitate d'ascorbyle.

Il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau, tout en étant stable dans une composition, ou bien alternativement qui possèdent une action renforcée de façon à pouvoir être utilisé en quantité plus faible, ce qui diminue considérablement les effets secondaires observés.

A cet égard la Demanderesse a de manière surprenante et inattendue découvert que certains dérivés esters de l'acide ascorbique présentaient une bonne activité dépigmentante, même à faible concentration, sans faire preuve de cytotoxicité. Ces dérivés présentent de plus l'avantage d'agir à la fois sur plusieurs composantes du mécanismes de la pigmentation. Ils agissent en inhibant la biosynthèse de la mélanine et en inhibant la dendricité des mélanocytes, diminuant ainsi la quantité de mélanine transférée aux kératinocytes voisins.

La présente invention a donc pour objet l'utilisation cosmétique d'au moins un composé de formule (I) : 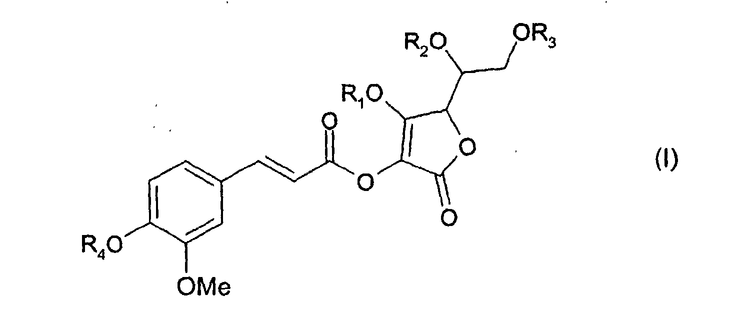 dans laquelle,
R₁, et R₄ représentent indépendamment un atome d'hydrogène, ou un groupement choisi parmi les groupements méthyle, éthyle, isopropyle, férulyle, benzyle, triméthylsilyle, triphénylsilyle, tertbutyldiméthylsilyle, acétyle, propanoyle, palmitoyle, lipoyle, benzoyle, SO₃H, et O=P(OH)OH, et CH₃NHCO- ,
R₂, et R₃ représentent indépendamment un atome d'hydrogène, ou un groupement choisi parmi les groupements méthyle, éthyle, isopropyle, férulyle, benzyle, triméthylsilyle, triphénylsilyle, tertbutyldiméthylsilyle, acétyle, propanoyle, palmitoyle, lipoyle, benzoyle, SO₃H, et O=P(OH)OH, et CH₃NHCO- , ou bien forment ensemble avec les oxygènes auxquels ils sont rattachés un groupement cyclique comportant de 5 à 6 chaînons, l'un au moins de ces deux groupements R₂ ou R₃ étant différent d'un atome d'hydrogène,
dans une composition, comme agent blanchissant de la peau et/ou des poils et/ou des cheveux.

Un aspect avantageux de l'invention concerne l'utilisation des composés de formule (I) pour lesquels R₁ représente un atome d'hydrogène.

Dans un autre aspect avantageux de l'invention, les composés de formule (I) utilisés sont ceux pour lesquels le groupement R₄ représente un atome d'hydrogène ou un groupement acétyle.

Dans des composés préférés de formule (I), les groupements R₂ et R₃ forment ensemble un groupement isopropylidène.

Un composé de formule (I) convenant particulièrement bien à la mise en oeuvre de la présente invention est l'ester 5-(2,2-diméthyl[1,3]dioxolan-4-yl)-4-hydroxy-2-oxo-2,5-dihydro-3-furanyle de l'acide 3-[(4-acétoxy-3-méthoxy)phényl]acrylique de formule (A) :

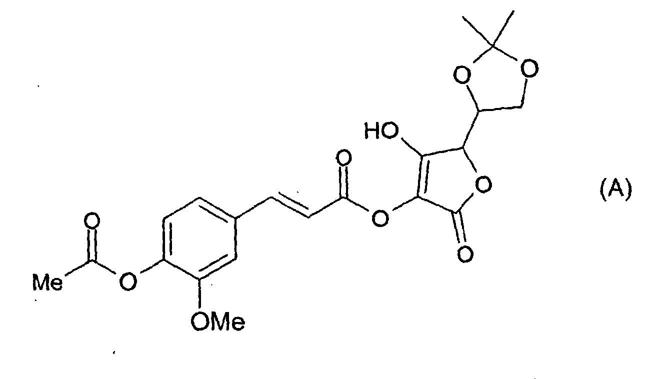

Un autre composé de formule (I) convenant particulièrement bien à la mise en oeuvre de la présente invention est l'ester 5-(2,2-diméthyl[1,3]dioxolan-4-yl)-4-hydroxy-2-oxo-2,5-dihydro-3-furanyle de l'acide 3-[(4-hydroxy-3-méthoxy)phényl]acrylique de formule (B) :

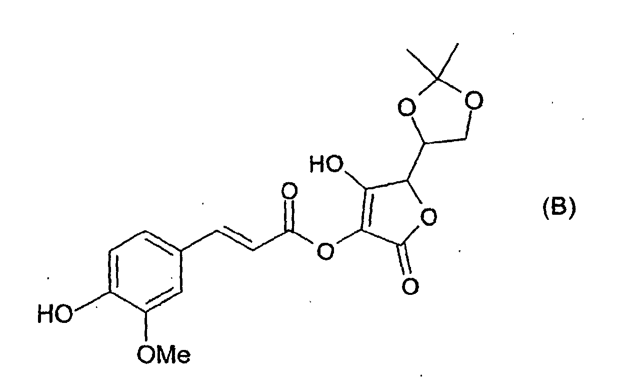

Les composés de formule (I) peuvent être obtenus par l'homme du métier par synthèse selon des méthodes usuelles, notamment en utilisant les procédés décrits dans la demande EP-0 664 290. En particulier, le composé A est décrit dans cette demande à l'étape 4 de l'exemple 1, et le composé C est décrit dans l'étape 5 de l'exemple 1.

La demande EP-A-1145710 décrit l'utilisation des composés selon les formules (A) et (B) pour augmenter la synthèse des céramides épidermiques.

La présente invention concerne aussi l'utilisation cosmétique d'au moins un composé de formule (I) tel que défini ci-dessus, dans une composition, en tant qu'agent anti-brunissement de la peau.

L'invention concerne également l'utilisation d'au moins un composé de formule (I) tel que défini ci-dessus pour la fabrication d'une composition dermatologique destinée à dépigmenter la peau et/ou les poils et/ou les cheveux.

L'invention a également pour objet un procédé cosmétique de blanchiment de la peau humaine, et/ou des poils et/ou des cheveux, comprenant l'application sur la peau et/ou les poils et/ou les cheveux, d'une composition cosmétique contenant un composé de formule (I) telle que définie ci-dessus.

La quantité de dérivés utilisable dans le cadre de l'invention dépend bien évidemment de l'effet recherché.
A titre d'exemple, cette quantité peut aller par exemple de 0,001% à 10% en poids, de préférence de 0,01% à 5% en poids, notamment de 0,1 à 2% en poids, par rapport au poids total de la composition.

La composition selon l'invention est notamment destinée à une application topique. Elle comprend par ailleurs un milieu physiologiquement acceptable, c'est-à-dire compatible avec la peau y compris le cuir chevelu, les muqueuses, les cheveux, les poils et/ou les yeux et peut constituer notamment une composition cosmétique ou dermatologique.

La composition peut alors comprendre tous les constituants usuellement employés dans l'application envisagée.
On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooing ou d'après-shampooing.

Cette composition peut constituer une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.

Dans un aspect avantageux de l'invention, les compositions utilisées peuvent comporter en plus au moins un agent desquamant, et/ou au moins un agent apaisant, et/ou au moins agent photoprotecteur organique et/ou au moins un agent photoprotecteur inorganique.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica; le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphea alba) de Seppic, un extrait du Pygeum, un extrait de Boswellia scrrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

Les agents photoprotecteurs organiques sont notamment choisis parmi les anthranilates ; les dérivés cinnamiques ; les dérivés de dibenzoylméthane; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA); les dérivés de méthylène bis-(hydroxyphényl benzotriazole) tels que décrits dans s les demandes US5,237,071, US5,166,355, GB2303549, DE 197 26 184 et EP893119 ; et les polymères filtres et silicones filtres tels que ceux décrits notamment dans la demande WO-93/04665 ; les dimères dérivés d'α-alkylstyrène tels que ceux décrits dans la demande de brevet DE19855649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple 1 : Mise en évidence de l'activité sur la mélanogénèse.

Un test biologique a mis en évidence l'activité dépigmentante des dérivés esters de l'acide ascorbique de formule (I).

L'effet modulateur sur la mélanogénèse des composés de formule (I) a été mesuré selon la méthode décrite dans la demande EP-0 993 826 déposée par la Demanderesse, ainsi que dans l'article de R. Schmidt, P. Krien et M. Régnier, Anal. Biochem., 235(2), 113-18, (1996).

Pour chaque composé testé, ont été déterminés :
- la cytotoxicité, en estimant l'incorporation de la leucine,
- l'activité inhibitrice sur la synthèse de la mélanine, en estimant le rapport de l'incorporation de thiouracile à l'incorporation de leucine, rapporté à 100 % du témoin (le témoin correspond au test réalisé sans composé à testé).
- L'IC50 qui correspond à la concentration micromolaire (µM) pour laquelle est observée 50% d'inhibition de la mélanogénèse.

Les résultats sont rassemblés dans le tableau suivant :

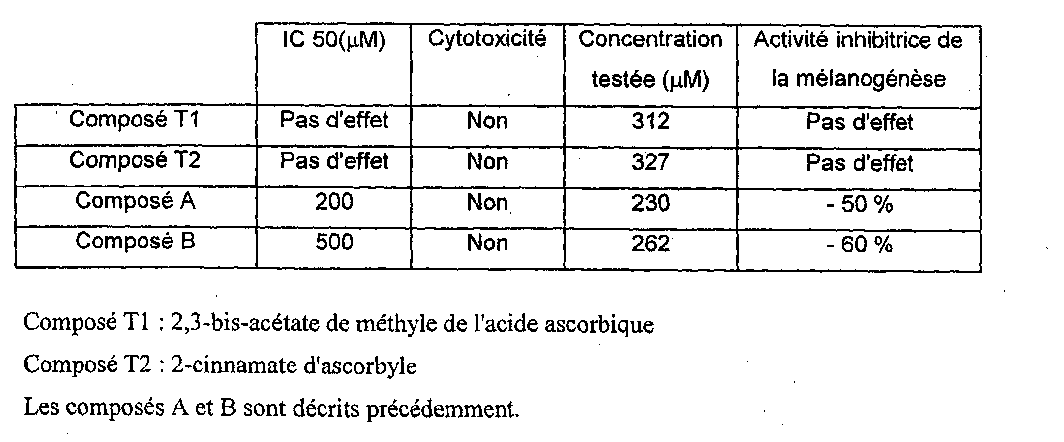

Les composés de formule (I) se montrent donc très efficaces pour inhiber la mélanogénèse, contrairement aux autres esters de l'acide ascorbique testés.

### Exemple 2 : Mise en évidence de l'activité sur la dendricité des mélanocytes :

Le but de ce test est de montrer l'effet des composés utilisés selon l'invention sur la modulation morphologique des mélanocytes.

### Méthode:

Les mélanocytes sont traitées dès l'ensemencement avec les composés selon l'invention pendant 7 jours. Les composés sont utilisés à une dose de 100 µg par ml.

### Observations:

Les mélanocytes sont marqués à l'aide de l'anticorps NKlbeteb (coloration verte) reconnaissant les mélanosomes à tout stade de maturation. Le noyau de la totalité des cellules, mélanocytes et kératinocytes, est coloré à l'aide de l'iodure de propidium (coloration rouge).

### Résultats:

- En absence des composés selon l'invention, les mélanocytes en co-cultures sont très dendritiques. Le pigment est visiblement transféré aux kératinocytes avoisinants.
- En présence des composés selon l'invention, la plupart des mélanocytes montre une dendricité réduite et certains mélanocytes deviennent bipolaires.
- Pour une concentration plus importante, la majorité des mélanocytes est bipolaire et la quantité de pigment transférée aux kératinocytes est diminuée.

Les observations sont résumées dans le tableau suivant :

| **Composé testé** | **Concentration testée (µM)** | **Dendricité** | **Transfert de pigmentation** |
|---|---|---|---|
| Témoin | - | Multipolaire | Important |
| Composé T1 | 312 | Multipolaire | Important |
| Composé T2 | 327 | Multipolaire | Important |
| Composé A | 230 | Bipolaire | Peu de pigmentation transférée |
| Composé B | 262 | Bipolaire | Peu de pigmentation transférée |

Pour le témoin le test a été mené sans composé selon l'invention.
Composé T1 : 2,3-bis-acétate de méthyle de l'acide ascorbique
Composé T2 : 2-cinnamate d'ascorbyle
Les composés A et B sont décrits précédemment. Pour ces derniers, il ressort des observations, que les mélanocytes deviennent bipolaires et que la quantité de pigment présente dans les kératinocytes est diminuée après traitement, en comparaison avec le témoin et les deux composés de référence T1 et T2.

### Exemple 3

On prépare une crème blanchissante de soin du visage de type émulsion huile-dans-eau, comprenant (% en poids) :
- ester 5-(2,2-diméthyl[1,3]dioxolan-4-yl)-4-hydroxy-2-oxo-2,5-dihydro-3-furanyle de l'acide 3-[(4-acétoxy-3-méthoxy) phényl]acrylique 0,005%
- stéarate de glycérol 2%
- polysorbate 60 (Tween 60 de ICI) 1%
- acide stéarique 1,4%
- triéthanolamine 0,7%
- carbomer 0,4%
- fraction liquide du beurre de karité 12%
- perhydrosqualène 12%
- antioxydant 0,05%
- parfum, conservateur qs
- eau qsp 100%

### Exemple 4

On prépare un gel dépigmentant pour la peau comprenant (% en poids) :
- ester 5-(2,2-diméthyl[1,3]dioxolan-4-yl)-4-hydroxy-2-oxo-2,5-dihydro-3-furanyle de l'acide 3-[(4-hydroxy-3-méthoxy) phényl]acrylique 2%
- hydroxypropylcellulose (Klucel H de Hercules) 1%
- antioxydant 0,05%
- isopropanol 40%
- parfum, conservateur qs
- eau qsp 100%

## Revendications

1. Utilisation cosmétique d'au moins un composé de formule (I) : 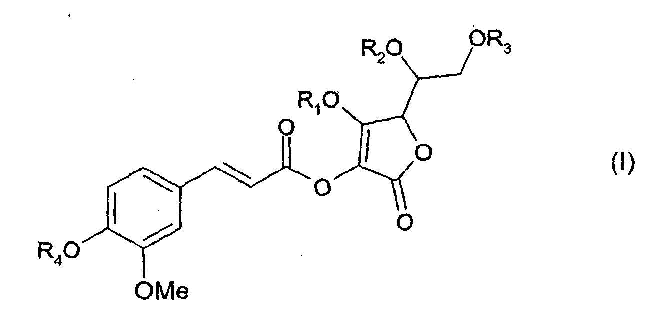 dans laquelle,
R₁, et R₄ représentent indépendamment un atome d'hydrogène, ou un groupement choisi parmi les groupements méthyle, éthyle, isopropyle, férulyle, benzyle, triméthylsilyle, triphénylsilyle, tertbutyldiméthylsilyle, acétyle, propanoyle, palmitoyle, lipoyle, benzoyle, SO₃H, et O=P(OH)OH, et CH₃NHCO-,
R₂, et R₃ représentent indépendamment un atome d'hydrogène, ou un groupement choisi parmi les groupements méthyle, éthyle, isopropyle, férulyle, benzyle, triméthylsilyle, triphénylsilyle, tertbutyldiméthylsilyle, acétyle, propanoyle, palmitoyle, lipoyle, benzoyle, SO₃H, et O=P(OH)OH, et CH₃NHCO- , ou bien forment ensemble avec les oxygènes auxquels ils sont rattachés un groupement cyclique comportant de 5 à 6 chaînons, l'un au moins de ces deux groupements R₂ ou R₃ étant différent d'un atome d'hydrogène,
dans une composition cosmétique, comme agent blanchissant de la peau et/ou des poils et/ou des cheveux.

2. Utilisation d'au moins un composé de formule (I) tel que défini dans la revendication 1, pour la fabrication d'une composition dermatologique destinée à dépigmenter la peau et/ou les poils et/ou les cheveux.

3. Utilisation selon l'une des revendications 1 et 2, **caractérisée en ce que** le groupement R₁ représente un atome d'hydrogène.

4. Utilisation selon l'une des revendications 1 à 3, **caractérisée en ce que** le groupement R₄ représente un atome d'hydrogène, ou un groupement acétyle.

5. Utilisation selon l'une des revendications 1 à 4, **caractérisée en ce que** les groupements R₂ et R₃ forment ensemble un groupement isopropylidène.

6. Utilisation selon l'une des revendications 1 à 5, **caractérisée en ce que** le composé de formule (I) est l'ester 5-(2,2-diméthyl[1,3]dioxolan-4-yl)-4-hydroxy-2-oxo-2,5-dihydro-3-furanyle de l'acide 3-[(4-acétoxy-3-méthoxy)phényl]acrylique.

7. Utilisation selon l'une des revendications 1 à 5, **caractérisée en ce que** le composé de formule (I) est l'ester 5-(2,2-diméthyl[1,3]dioxolan-4-yl)-4-hydroxy-2-oxo-2,5-dihydro-3-furanyle de l'acide 3-[(4-hydroxy-3-méthoxy)phényl]acrylique.

8. Utilisation cosmétique d'au moins un composé de formule (I) tel que défini dans la revendications 1, dans une composition, en tant qu'agent anti-brunissement de la peau.

9. Procédé cosmétique de blanchiment de la peau humaine, et/ou des poils et/ou des cheveux, comprenant l'application sur la peau et/ou les poils et/ou les cheveux, d'une composition cosmétique contenant au moins un composé de formule (I) telle que définie dans la revendication 1.

## Patentansprüche

1. Kosmetische Verwendung mindestens einer Verbindung der Formel (I): 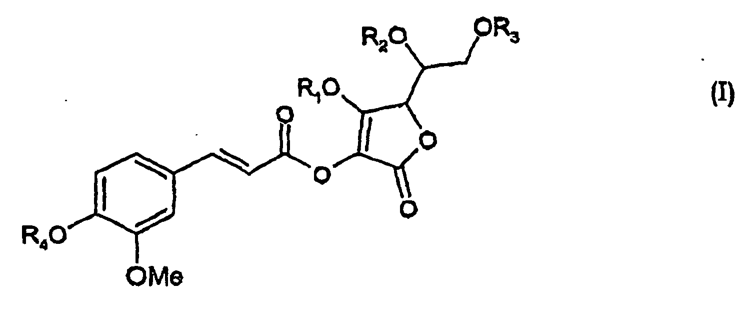 worin bedeuten:
R₁ und R₄ unabhängig voneinander ein Wasserstoffatom oder eine Gruppe, die unter Methyl, Ethyl, Isopropyl, Ferulyl, Benzyl, Trimethylsilyl, Triphenylsilyl, *t*-Butyldimethylsilyl, Acetyl, Propanoyl, Palmitoyl, Lipoyl, Benzoyl, SO₃H, O=P(OH)OH und CH₃NHCO- ausgewählt ist,
R₂ und R₃ unabhängig voneinander ein Wasserstoffatom oder eine Gruppe, die unter Methyl, Ethyl, Isopropyl, Ferulyl, Benzyl, Trimethylsilyl, Triphenylsilyl, *t*-Butyldimethylsilyl, Acetyl, Propanoyl, Palmitoyl, Lipoyl, Benzoyl, SO₃H, O=P(OH)OH und
CH₃NHCO- ausgewählt ist, oder sie bilden gemeinsam mit den Sauerstoffatomen, an die sie gebunden sind, eine cyclische Gruppe, die 5 bis 6 Glieder aufweist, wobei mindestens eine der beiden Gruppen R₂ oder R₃ von Wasserstoff verschieden ist,
in einer Zusammensetzung als Wirkstoff zum Aufhellen der Haut und/oder der Körperhaare und/oder der Haare.

2. Verwendung mindestens einer Verbindung der Formel (I) nach Anspruch 1 zur Herstellung einer dermatologischen Zusammensetzung, die dazu vorgesehen ist, die Haut und/oder Körperhaare und/oder die Haare zu depigmentieren.

3. Verwendung nach einem der Ansprüche 1 und 2, **dadurch gekennzeichnet, dass** die Gruppe R₁ ein Wasserstoffatom bedeutet.

4. Verwendung nach einem der Ansprüche 1 bis 3, **dadurch gekennzeichnet, dass** die Gruppe R₄ ein Wasserstoffatom oder eine Acetylgruppe bedeutet.

5. Verwendung nach einem der Ansprüche 1 bis 4, **dadurch gekennzeichnet, dass** die Gruppen R₂ und R₃ gemeinsam eine Isopropylidengruppe bilden.

6. Verwendung nach einem der Ansprüche 1 bis 5, **dadurch gekennzeichnet, dass** die Verbindung der Formel (I) der 3-[(4-Acetoxy-3-methoxy)phenyl]-acrylsäure-5-(2,2-dimethyl[1,3]-dioxolan-4-yl)-4-hydroxy-2-oxo-2,5-dihydro-3-furanylester ist.

7. Verwendung nach einem der Ansprüche 1 bis 5, **dadurch gekennzeichnet, dass** die Verbindung der Formel (I) der 3-[(4-Hydroxy-3-methoxy)phenyl]-acrylsäure-5-(2,2-dimethyl[1,3]-dioxolan-4-yl)-4-hydroxy-2-oxo-2,5-dihydro-3-furanylester ist.

8. Kosmetische Verwendung mindestens einer Verbindung der Formel (I) nach Anspruch 1 in einer Zusammensetzung als Wirkstoff, der der Braunfärbung der Haut entgegenwirkt.

9. Kosmetisches Verfahren zum Aufhellen der menschlichen Haut und/oder der Körperhaare und/oder der Haare, das umfasst, auf die Haut und/oder die Körperhaare und/oder die Haare eine kosmetische Zusammensetzung aufzutragen, die mindestens eine in Anspruch 1 definierte Verbindung der Formel (I) enthält.

## Claims

1. Cosmetic use of at least one compound of formula (I): 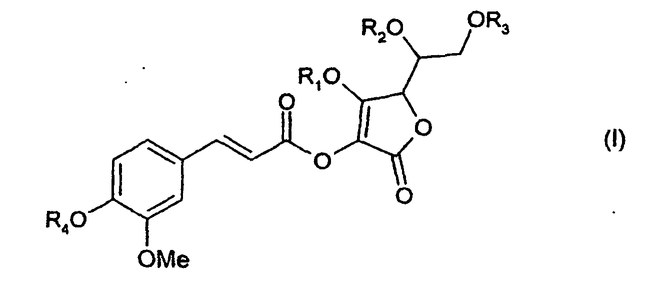 in which,
R₁ and R₄ independently represent a hydrogen atom or a group chosen from methyl, ethyl, isopropyl, ferulyl, benzyl, trimethylsilyl, triphenylsilyl, tert-butyldimethylsilyl, acetyl, propanoyl, palmitoyl, lipoyl, benzoyl, SO₃H, O=P(OH)OH and CH₃NHCO- groups,
R₂ and R₃ independently represent a hydrogen atom or a group chosen from methyl, ethyl, isopropyl, ferulyl, benzyl, trimethylsilyl, triphenylsilyl, tert-butyldimethylsilyl, acetyl, propanoyl, palmitoyl, lipoyl, benzoyl, SO₃H, O=P(OH)OH and CH₃NHCO- groups, or alternatively form, together with the oxygens to which they are attached, a 5- or 6-membered cyclic group, at least one of these two groups R₂ or R₃ being other than a hydrogen atom,
in a cosmetic composition, as an agent for bleaching the skin and/or head hair and/or other hairs.

2. Use of at least one compound of formula (I) as defined in Claim 1, for the manufacture of a dermatological composition for depigmenting the skin and/or head hair and/or other hairs.

3. Use according to either of Claims 1 and 2, **characterized in that** the group R₁ represents a hydrogen atom.

4. Use according to one of Claims 1 to 3, **characterized in that** the group R₄ represents a hydrogen atom or an acetyl group.

5. Use according to one of Claims 1 to 4, **characterized in that** the groups R₂ and R₃ together form an isopropylidene group.

6. Use according to one of Claims 1 to 5, **characterized in that** the compound of formula (I) is the ester 5-(2,2-dimethyl[1,3]dioxolan-4-yl)-4-hydroxy-2-oxo-2,5-dihydro-3-furanyl 3-[(4-acetoxy-3-methoxy)-phenyl]acrylate.

7. Use according to one of Claims 1 to 5, **characterized in that** the compound of formula (I) is the ester 5-(2,2-dimethyl[1,3]dioxolan-4-yl)-4-hydroxy-2-oxo-2,5-dihydro-3-furanyl 3-[(4-hydroxy-3-methoxy)-phenyl]acrylate.

8. Cosmetic use of at least one compound of formula (I) as defined in Claim 1, in a composition, as an anti-tanning agent for the skin.

9. Cosmetic process for bleaching human skin and/or head hair and/or other hairs, comprising the application to the skin and/or head hair and/or other hairs of a cosmetic composition containing at least one compound of formula (I) as defined in Claim 1.
